# EUROPEAN PATENT SPECIFICATION

(11) **EP 3 493 866 B1**
(45) Date of publication and mention of the grant of the patent: **17.06.2020**
(21) Application number: 17754200.8
(22) Date of filing: 19.07.2017
(51) Int. Cl.: A61M 5/44

(54) **SELF-HEATED INTRAVENOUS APPARATUS**
SELBSTHEIZENDE INTRAVENÖSE VORRICHTUNG
APPAREIL INTRAVEINEUX AUTO-CHAUFFANT

(30) Priority: 04.08.2016 CH 10092016
(43) Date of publication of application: 12.06.2019
(73) Proprietor: Frasnelli, Andreas, 3904 Naters (CH)
(72) Inventor: Frasnelli, Andreas, 3904 Naters (CH)
(74) Representative: P&TS SA (AG, Ltd.)
(86) International application number: PCT/IB2017/054348
(87) International publication number: WO 2018/025107

(56) References cited:
- WO-A2-03/059414
- US-A1- 2011 194 846

## Description

### Field of the invention

The present invention concerns a self-heated intravenous apparatus, a method to manufacture a self-heated intravenous apparatus and a method to prepare an intravenous therapy.

### Description of related art

The undercooling of injured persons before arriving at the hospital happens often, in particular where recovery is cumbersome like in the mountains. One of the possible counter measures is an intravenous therapy, also called IV therapy or infusion, with heated infusion liquid. Infusion liquid shall be the liquid for infusion in the vein. In the following different state of the art methods to heat the infusion liquid are presented.

One possibility is to heat up the infusion liquid electrically. In US2014270738 a heating device heats up the liquid in the infusion bag, i.e. the bag containing the infusion liquid. However, this solution has the disadvantage that it takes quite a while to heat up the complete infusion liquid in the infusion bag and there might be the risk of strong temperature fluctuations within the infusion bag. US2009319011 and CN2724709Y propose instead electrical devices for heating the infusion liquid directly in the intravenous tube so that the heating effect can be obtained immediately. However, electrical devices have the disadvantage to be heavy and space-consuming and to need electric power supply or heavy batteries. In particular, in flying ambulances or in alpine rescue service space, capacity for weight or electricity is not available. In addition, electrical devices need maintenance in order to avoid a failure during a rescue mission. WO03059414 discloses features falling under the preamble of claim 1. The invention is defined in claims 1, 11 and 14. Further embodiments of the invention are defined in the dependent claims.

An alternative to electrical heating devices are heating devices working with exothermic reactions.

US2013226087, CN203370168U and WO080177456 disclose a disposable heating device with two liquids which create heat by an exothermic reaction when they are mixed. The heat is transferred to the IV line or infusion tube. However, such disposable heating devices are also heavy and space consuming. In addition, there is also a risk of initiating the exothermic reaction by mistake during the transport.

CN2261254Y discloses an intravenous tube with an outer wall comprising a catalyst layer and a heating layer separated by a septum. By damaging the septum, the catalyst can enter the heating layer and cause an exothermic reaction to heat the infusion liquid in the intravenous tube. Also this solution has a risk of initiating the exothermic reaction by mistake during the transport.

CN201744029U, CN101670135A and US2013298903A disclose heating layers adhesively connected to the intravenous tube or the infusion bag. The exothermic reaction of the heating layer for heating the infusion liquid is activated by air, when a barrier layer is removed. This solution has the disadvantage that the heating effect can fluctuate depending on where and how the heating layer is arranged on the infusion bag or the intravenous tube by the user.

Another disadvantage of all of the described solutions is that they require extra time of the rescue team for initiating the heat reaction or for mounting the heating device.

### Brief summary of the invention

It is an object of the invention to overcome the disadvantages of the state of the art and to find a solution which does not need extra time for the rescue team, does not need a power supply, does not need maintenance and reduces extra weight and space.

According to one embodiment, the object is achieved by a self-heated intravenous apparatus comprising an intravenous tube with a tube wall. A heat generation layer is coated on the tube wall configured to perform an exothermic reaction activated by oxygen and/or water for heating an infusion liquid conducted in the intravenous tube.

According to one embodiment, the object is achieved by a method to manufacture a self-heated intravenous apparatus comprising the steps providing an intravenous tube with an outer wall and coating a heat generation layer on the outer wall, wherein the heat generation layer is configured to perform an exothermic reaction activated by oxygen and/or water for heating an infusion liquid conducted in the intravenous tube.

Since oxygen and/or water is/are present in the ambient air and the heat generation layer is already coated on the intravenous tube, no extra steps are needed by the rescue team for activating the exothermic reaction to heat the infusion liquid, when the intravenous tube is used in an emergency situation, and the handling of the intravenous tube remains exactly the same as standard intravenous tubes. This saves time for other rescue steps of the rescue team and avoids learning complicated and error-prone protocols for heating infusion liquids. In addition, the present solution does only require minimal additional weight or space compared to a classical intravenous tube.

According to one embodiment, the object is achieved by a self-heated intravenous apparatus comprising an intravenous tube, a heat generation means configured to perform an exothermic reaction activated by oxygen and/or water of ambient air for heating an infusion liquid conducted in the intravenous tube and a hermetically sealed bag containing the intravenous tube with the heat generation means, wherein the heat generation means is activated by opening the hermetically sealed bag.

According to one embodiment, the object is achieved by a method for preparing an intravenous therapy comprising the step of opening an hermetically sealed bag containing an intravenous tube with a heat generation means so that oxygen and/or water of ambient air contacts the heat generation layer and activates an exothermic reaction of the heat generation means for heating an infusion liquid conducted through the intravenous tube.

This embodiment has the advantage that the heat generation means is activated by opening the sterile bag of the intravenous tube by ambient air and/or water. Therefore, no extra steps are needed by the rescue team for activating the exothermic reaction to heat the infusion liquid, when the intravenous tube is used in an emergency situation. This saves time for other rescue steps of the rescue team and avoids learning complicated and error-prone protocols for heating infusion liquids. In addition, the present solution does only require minimal additional weight or space compared to a classical intravenous tube.

The dependent claims refer to further advantageous embodiments.

### Brief Description of the Drawings

The invention will be better understood with the aid of the description of an embodiment given by way of example and illustrated by the figures, in which:
Fig. 1 shows a first embodiment of the self-heated intravenous apparatus.
Fig. 2 shows a cross-sectional view of the intravenous tube.
Fig. 3 shows a second embodiment of the self-heated intravenous apparatus.

### Detailed Description of possible embodiments of the Invention

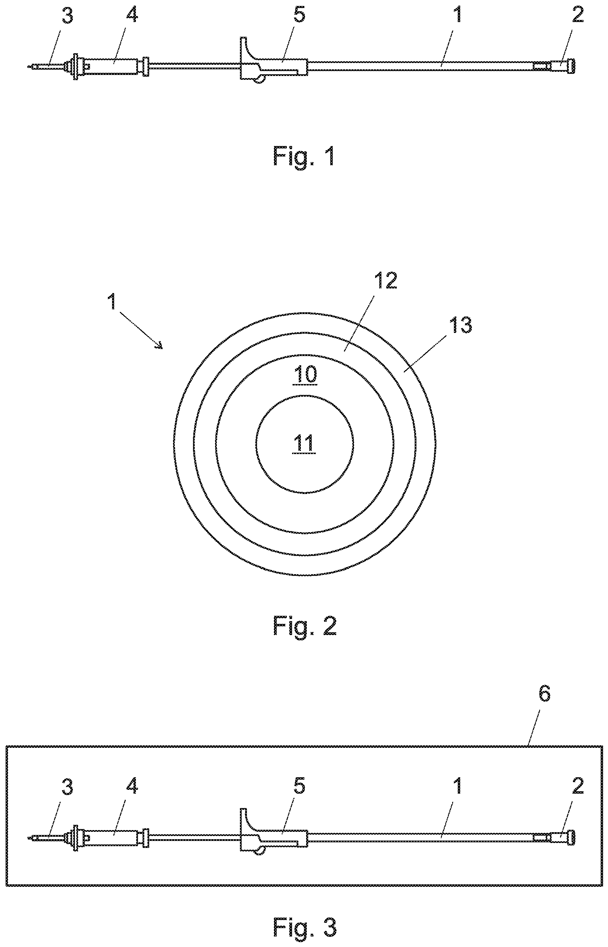

Fig. 1 shows a first embodiment of a self-heated intravenous apparatus.

The self-heated intravenous apparatus comprises an intravenous tube 1 and a heat generation means.

The intravenous tube 1 is configured to conduct an infusion liquid, preferably from an infusion bag to an intravenous access device. The intravenous tube 1 is preferable flexible. The intravenous tube 1 is preferable transparent to see the liquid in the intravenous tube 1. The tube 1 is formed by a tube wall 10 as shown in Fig. 2. The tube wall 10 provides a conduit 11 for conducting the infusion liquid along the longitudinal axis of the intravenous tube 1. The tube wall 10 has preferably a round cross-section. The material of the tube wall is preferably flexible and/or preferable transparent.

The heat generation means is configured to perform an exothermic reaction, when in contact with ambient air, oxygen and/or water, preferably when in contact with oxygen and/or water from ambient air. The heat generation means is arranged such that, once activated, it transfers the generated heat to the tube wall 10 of the intravenous tube 1 in order to heat the infusion liquid conducted in the conduit 11 of the intravenous tube 1.

In a preferred embodiment, the heat generation means is a heat generation layer 12 coated on the outer side of the tube wall 10. This achieves an optimal heat transfer from the heat generation means to the conduit 11. Preferably, the heat generation layer 12 is arranged (uniformly) 360° around the circumference of the intravenous tube 1 or of the tube wall 10 in order to heat the infusion liquid uniformly from all sides. However, also embodiments are possible, where the heat generation layer 12 is coated only on portions of the circumference of the intravenous tube 1. Preferably, the heat generation layer 12 is coated along at least 50%, preferably at least 60%, preferably at least 70%, preferably at least 80%, preferably at least 90% or 100% of the length of the intravenous tube 1. This maximises the amount of heat transferable to the infusion liquid.

In a preferred embodiment, a heat insulating layer 13 is coated on/around the heat generation layer 12. The heat insulating layer 13 is configured for reducing or stopping heat dissipation from the heat generation layer 12 to the ambience and/or for transporting air, oxygen and/or water to the heat generation layer 12. The latter function can be achieved by holes or pores in the heat insulating layer 13 allowing air, oxygen and/or water be transported through the heat insulating layer 13.

The heat generation layer 12 coated on the tube wall 10 is a preferred embodiment of the heat generation means. However, also other heat generation means activated by air, oxygen and/or water can be used to transfer heat to the intravenous tube 1, e.g. the heat generation layer 12 can be glued or adhesively bonded on the tube wall 12 or the heat generation means can also have different form than a layer.

Preferably, the self-heated intravenous apparatus comprises a barrier means for avoiding the heat generation layer 12 or heat generation means to contact ambient air, oxygen or water so that the exothermic reaction of the heat generation layer 12 is not activated by mistake.

The barrier means is preferably a hermetically sealed bag 6 as shown in Fig. 6 containing the intravenous tube with the heat generation layer 12. The hermetically sealed bag 6 fulfils preferably two functions. First, it keeps the intravenous tube 1 sterile. Second, it avoids activation of the heat generation layer 12. Thus, the rescue team has just to bring the hermetically sealed bag with the intravenous tube 1. The team opens the bag and can start immediately the intravenous therapy. The exothermic reaction of the heat generation layer 12 on the intravenous tube 1 is activated without any extra steps. Also the safety is increased, because the number of potential errors is reduced. Due to the fixed arrangement of the heat generation layer 12 on the intravenous tube 1, the transferred heat is not reduced by placing the heat generation layer by a user in a wrong position or in a bad way. Everything needed is already in the hermetically sealed bag 6 and the necessary equipment weight and dimension is reduced compared to the state of the art solutions for heating the infusion liquid.

The barrier means can comprise alternatively or additionally an adhesive barrier layer around the intravenous tube 1 which blocks the contact of the heat generation layer 12 with any surrounding air, oxygen and/or water. In this case, the user has (additionally to opening the bag 6) to remove the barrier layer around the intravenous tube 1 to activate the exothermic reaction.

The self-heated intravenous apparatus can comprise an inlet means for inserting the infusion liquid in the intravenous tube 1. An example for an inlet means is a needle 3 to access a liquid of an infusion bag. However, the inlet means can also be a port to connect another intravenous tube or other intravenous equipment.

The self-heated intravenous apparatus can comprise an outlet means for letting out the infusion liquid from the intravenous tube 1. An example for an outlet means is a port 2 to connect the intravenous tube 1 to an intravenous access device, e.g. a catheter, or to any other intravenous equipment. However, the outlet means can also be other means like directly a catheter.

The self-heated intravenous apparatus can comprise other intravenous equipment along the intravenous tube 1 like a roller clamp 5 to regulate the flow rate of the infusion liquid, drip chamber 4 to remove air bubbles from the liquid, a back check valve, a port as inlet for a second infusion liquid from a second infusion bag, other intravenous equipment or any combination or sub-combination of those.

One embodiment to manufacture such a self-heated intravenous apparatus could be to use a standard intravenous tube as available on the market. A heat generation layer 12 as described above can be coated, glued or otherwise disposed on the tube wall 10 of the intravenous tube 1. Preferably, the manufacturing environment does not contain the activation substance air, oxygen and/or water in order to avoid an activation of the heat generation layer 12. Alternatively, the manufacture environment could contain any atmosphere which does not activate the heat generation layer 12. In a preferred embodiment, a heat insulating layer 13 is provided around the heat generation layer 12. The intravenous tube 1 with the heat generation layer 12 is packed in a hermetically sealed bag 6. Either the manufacturing atmosphere is sterile and/or the intravenous tube 1 is sterilized before being packed in the hermetically sealed bag 6 so that the intravenous tube 1 with the heat generation layer 12 in the hermetically sealed bag 6 is sterile and ready for use with a patient. To avoid the activation of the heat generation layer 12, it is advisable to reduce the content of the activation substance air, oxygen and/or water in the hermetically sealed bag 6 such that an activation of the exothermic reaction of the heat generation layer 12 is avoided. This could be realised by an inert gas or vacuum in the hermetically sealed bag 6. In a preferred embodiment, the heat generation layer 12 is provided on the intravenous tube 1 before the intravenous tube 1 with the heat generation layer 12 is packed in the hermetically sealed bag 6.

## Claims

1. Self-heated intravenous apparatus comprising
an intravenous tube (1) with a tube wall (10);
**characterized by**
a heat generation layer (12) coated on the tube wall (10) configured to perform an exothermic reaction activated by oxygen and/or water for heating an infusion liquid conducted in the intravenous tube (1).

2. Self-heated intravenous apparatus according to claim 1, comprising a barrier means (6) for avoiding the activation of the exothermic reaction of the heat generation layer (12) by oxygen and/or water.

3. Self-heated intravenous apparatus according to claim 2, wherein the barrier means (6) is configured to be opened to activate the exothermic reaction of the heat generation layer (12).

4. Self-heated intravenous apparatus according to claim 2 or 3, wherein the barrier means (6) comprises a hermetically sealed bag containing the intravenous tube (1) with the heat generation layer (12).

5. Self-heated intravenous apparatus according to claim 4, wherein the heat generation layer (12) is configured to be activated by opening the sealed bag (6) and by bringing the heat generation layer (12) in contact with oxygen and/or water from ambient air.

6. Self-heated intravenous apparatus according to one of claims 2 to 5, wherein the barrier means (6) comprises an adhesive barrier layer attached around the intravenous tube (1) for blocking the contact of the heat generation layer (12) with ambient air, oxygen and/or water.

7. Self-heated intravenous apparatus according to one of claims 1 to 6, wherein the heat layer (12) is configured such that the exothermic reaction is activated by oxygen and/or water from ambient air.

8. Self-heated intravenous apparatus according to one of claims 1 to 7, comprising a heat insulating layer (13) on the heat generation layer (12) configured for stopping heat dissipation from the heat generation layer (12) to the ambience and for transporting oxygen and/or water to the heat generation layer (12).

9. Self-heated intravenous apparatus according to one of claims 1 to 8, wherein the outer wall (10) of the intravenous tube (1) is made of flexible material.

10. Self-heated intravenous apparatus according to one of claims 1 to 9, comprising an inlet means (3) at one end of the intravenous tube (1) to be connected to an infusion bag and an outlet means (2) at the other end of the intravenous tube (1) to be connected to an intravenous access device.

11. Method to manufacture a self-heated intravenous apparatus comprising the steps:
providing an intravenous tube (1) with an outer wall (10);
coating a heat generation layer (12) on the outer wall (10),
wherein the heat generation layer (12) is configured to perform an exothermic reaction activated by oxygen and/or water for heating an infusion liquid conducted in the intravenous tube (1).

12. Method according to claim 11, comprising the step of providing a barrier means (6) to avoid activation of the exothermic reaction of the heat generation layer (12) by oxygen and/or water.

13. Method according to claim 12, wherein the step of providing a barrier means (6) comprises to put the intravenous tube (1) with the heat generation layer (12) in a hermetically sealed bag, whose content of air and/or water is low enough for not activating the exothermic reaction of the heat generation layer (12).

14. Method for preparing an intravenous therapy, comprising the following steps:
opening an hermetically sealed bag (6) containing an apparatus according to claim 1 so that oxygen and/or water of ambient air contacts the heat generation layer (12) and activates an exothermic reaction of the heat generation layer (12) for heating an infusion liquid conducted through the intravenous tube (1).

## Patentansprüche

1. Selbstheizende intravenöse Vorrichtung, umfassend einen intravenösen Schlauch (1) mit einer Schlauchwand (10);
**gekennzeichnet durch**
eine Wärmeerzeugungsschicht (12), die als Beschichtung auf die Schlauchwand (10) aufgebracht ist und ausgelegt ist, um eine exotherme Reaktion durchzuführen, die durch Sauerstoff und/oder Wasser aktiviert wird, um eine Infusionsflüssigkeit zu erwärmen, die durch einen intravenösen Schlauch (1) geleitet wird.

2. Selbstheizende intravenöse Vorrichtung nach Anspruch 1, umfassend eine Barriere (6), um die Aktivierung der exothermen Reaktion der Wärmeerzeugungsschicht (12) durch Sauerstoff und/oder Wasser zu vermeiden.

3. Selbstheizende intravenöse Vorrichtung nach Anspruch 2, wobei die Barriere (6) ausgelegt ist, um geöffnet zu werden, um die exotherme Reaktion der Wärmeerzeugungsschicht (12) zu aktivieren.

4. Selbstheizende intravenöse Vorrichtung nach Anspruch 2 oder 3, wobei die Barriere (6) einen hermetisch versiegelten Beutel umfasst, der den intravenösen Schlauch (1) mit der Wärmeerzeugungsschicht (12) enthält.

5. Selbstheizende intravenöse Vorrichtung nach Anspruch 4, wobei die Wärmeerzeugungsschicht (12) ausgelegt ist, um aktiviert zu werden, indem der versiegelte Beutel (6) geöffnet wird und die Wärmeerzeugungsschicht (12) in Kontakt mit Sauerstoff und/oder Wasser aus Umgebungsluft gebracht wird.

6. Selbstheizende intravenöse Vorrichtung nach einem der Ansprüche 2 bis 5, wobei die Barriere (6) eine haftende Barriereschicht umfasst, die um den intravenösen Schlauch (1) herum angebracht ist, um den Kontakt der Wärmeerzeugungsschicht (12) mit Umgebungsluft, Sauerstoff und/oder Wasser zu blockieren.

7. Selbstheizende intravenöse Vorrichtung nach einem der Ansprüche 1 bis 6, wobei die Wärmeerzeugungsschicht (12) so ausgelegt ist, dass die exotherme Reaktion durch Sauerstoff und/oder Wasser aus Umgebungsluft aktiviert wird.

8. Selbstheizende intravenöse Vorrichtung nach einem der Ansprüche 1 bis 7, umfassend eine Wärmeisolierschicht (13) auf der Wärmeerzeugungsschicht (12), die ausgelegt ist, um die Wärmeableitung aus der Wärmeerzeugungsschicht (12) an die Umgebung zu stoppen und um Sauerstoff und/oder Wasser an die Wärmeerzeugungsschicht (12) zu transportieren.

9. Selbstheizende intravenöse Vorrichtung nach einem der Ansprüche 1 bis 8, wobei die Außenwand (10) des intravenösen Schlauchs (1) aus flexiblem Material gefertigt ist.

10. Selbstheizende intravenöse Vorrichtung nach einem der Ansprüche 1 bis 9, umfassend einen Zugang für Einlass (3) an einem Ende des intravenösen Schlauchs (1) zum Verbinden mit einem Infusionsbeutel und einem Zugang für Auslass (2) an dem anderen Ende des intravenösen Schlauchs (1) zum Verbinden mit einemvenösen Zugang.

11. Verfahren zum Fertigen einer selbstheizenden intravenösen Vorrichtung, umfassend die Schritte:
Bereitstellen eines intravenösen Schlauchs (1) mit einer Außenwand (10);
Beschichten der Außenwand (10) mit einer Wärmeerzeugungsschicht (12), wobei die Wärmeerzeugungsschicht (12) ausgelegt ist, um eine exotherme Reaktion durchzuführen, die durch Sauerstoff und/oder Wasser aktiviert wird, um eine Infusionsflüssigkeit zu erwärmen, die durch den intravenösen Schlauch (1) geleitet wird.

12. Verfahren nach Anspruch 11, umfassend den Schritt des Bereitstellens einer Barriere (6), um die Aktivierung der exothermen Reaktion der Wärmeerzeugungsschicht (12) durch Sauerstoff und/oder Wasser zu vermeiden.

13. Verfahren nach Anspruch 12, wobei der Schritt des Bereitstellens einer Barriere (6) bedeutet, dass der intravenöse Schlauch (1) mit der Wärmeerzeugungsschicht (12) in einen hermetisch versiegelten Beutel gegeben wird, dessen Gehalt an Luft und/oder Wasser hinreichend niedrig ist, um die exotherme Reaktion der Wärmeerzeugungsschicht (12) nicht zu aktivieren.

14. Verfahren zum Vorbereiten einer intravenösen Therapie, umfassend die folgenden Schritte:
Öffnen eines hermetisch versiegelten Beutels (6), der eine Vorrichtung nach Anspruch 1 enthält, so dass Sauerstoff und/oder Wasser aus Umgebungsluft die Wärmeerzeugungsschicht (12) kontaktieren und
eine exotherme Reaktion der Wärmeerzeugungsschicht (12) aktivieren, um eine Infusionsflüssigkeit zu erwärmen, die durch den intravenösen Schlauch (1) geleitet wird.

## Revendications

1. Appareil intraveineux autochauffant comprenant
un tube intraveineux (1) doté d'une paroi (10) de tube ;
**caractérisé par**
une couche de production de chaleur (12) revêtue sur la paroi (10) de tube conçue pour réaliser une réaction exothermique activée par l'oxygène et/ou l'eau afin de chauffer un liquide de perfusion conduit dans le tube intraveineux (1).

2. Appareil intraveineux autochauffant selon la revendication 1, comprenant un moyen de barrière (6) pour éviter l'activation de la réaction exothermique de la couche de production de chaleur (12) par l'oxygène et/ou l'eau.

3. Appareil intraveineux autochauffant selon la revendication 2, dans lequel le moyen de barrière (6) est conçu pour être ouvert afin d'activer la réaction exothermique de la couche de production de chaleur (12).

4. Appareil intraveineux autochauffant selon la revendication 2 ou 3, dans lequel le moyen de barrière (6) comprend une poche scellée hermétiquement contenant le tube intraveineux (1) avec la couche de production de chaleur (12).

5. Appareil intraveineux autochauffant selon la revendication 4, dans lequel la couche de production de chaleur (12) est conçue pour être activée en ouvrant la poche scellée (6) et en amenant la couche de production de chaleur (12) en contact avec l'oxygène et/ou l'eau provenant de l'air ambiant.

6. Appareil intraveineux autochauffant selon l'une des revendications 2 à 5, dans lequel le moyen de barrière (6) comprend une couche barrière adhésive fixée autour du tube intraveineux (1) pour bloquer le contact de la couche production de chaleur (12) avec l'air ambiant, l'oxygène et/ou l'eau.

7. Appareil intraveineux autochauffant selon l'une des revendications 1 à 6, dans lequel la couche de production de chaleur (12) est conçue de sorte que la réaction exothermique soit activée par l'oxygène et/ou l'eau provenant de l'air ambiant.

8. Appareil intraveineux autochauffant selon l'une des revendications 1 à 7, comprenant une couche d'isolation thermique (13) sur la couche de production de chaleur (12) conçue pour arrêter la dissipation de chaleur depuis la couche de production de chaleur (12) vers le milieu environnant et pour transporter l'oxygène et/ou l'eau vers la couche de production de chaleur (12).

9. Appareil intraveineux autochauffant selon l'une des revendications 1 à 8, dans lequel la paroi externe (10) du tube intraveineux (1) est en matériau souple.

10. Appareil intraveineux autochauffant selon l'une des revendications 1 à 9, comprenant un accès pour l'entrée (3) au niveau d'une extrémité du tube intraveineux (1) à raccorder à une poche de perfusion et un accès pour la sorite (2) au niveau de l'autre extrémité du tube intraveineux (1) à raccorder à un dispositif d'accès intraveineux.

11. Procédé de fabrication d'un appareil intraveineux autochauffant comportant les étapes consistant à :
fournir un tube intraveineux (1) doté d'une paroi externe (10) ;
revêtir une couche de production de chaleur (12) sur la paroi externe (10), la couche de production de chaleur (12) étant conçue pour réaliser une réaction exothermique activée par l'oxygène et/ou l'eau afin de chauffer un liquide de perfusion conduit dans le tube intraveineux (1).

12. Procédé selon la revendication 11, comprenant l'étape consistant à fournir un moyen de barrière (6) pour éviter l'activation de la réaction exothermique de la couche de production de chaleur (12) par l'oxygène et/ou l'eau.

13. Procédé selon la revendication 12, dans lequel l'étape consistant à fournir un moyen de barrière (6) consiste à placer le tube intraveineux (1) avec la couche de production de chaleur (12) dans une poche scellée hermétiquement, dont la teneur en air et/ou eau est suffisamment basse pour ne pas activer la réaction exothermique de la couche de production de chaleur (12).

14. Procédé de préparation d'un traitement par voie intraveineuse, comprenant les étapes suivantes consistant à :
ouvrir une poche scellée hermétiquement (6) contenant un appareil selon la revendication 1 de sorte que l'oxygène et/ou l'eau de l'air ambiant entrent en contact avec la couche de production de chaleur (12) et active une réaction exothermique de la couche de production de chaleur (12) afin de chauffer un liquide de perfusion conduit à travers le tube intraveineux (1).
